# EUROPÄISCHE PATENTSCHRIFT

(11) **EP 0 567 783 B1**
(45) Veröffentlichungstag und Bekanntmachung des Hinweises auf die Patenterteilung: **15.05.1996**
(21) Anmeldenummer: 93104999.3
(22) Anmeldetag: 26.03.1993
(51) Int. Cl.: A61F 5/04

(54) **Sprunggelenkorthese**
Angle joint orthosis
Appareil orthopédique pour la jambe et le pied

(30) Priorität: 30.04.1992 DE 9205791 U
(43) Veröffentlichungstag der Anmeldung: 03.11.1993
(73) Patentinhaber: Otto Bock Orthopädische Industrie Besitz- und Verwaltungs-KG, D-37115 Duderstadt (DE)
(72) Erfinder: Grifka, Joachim, Dr. med., W-4630 Bochum (DE)
(74) Vertreter: Gramm, Werner, Prof., Dipl.-Ing.

(56) Entgegenhaltungen:
- CH-A- 607 614
- DE-A- 2 259 945
- DE-B- 2 808 968
- FR-A- 2 354 087
- US-A- 3 916 886
- US-A- 5 022 390
- US-A- 5 121 742

## Beschreibung

Die Erfindung betrifft eine Sprunggelenkorthese mit einer dorsalen, sich über den Rückfuß erstreckenden Beinschale, die über Gurtungen oder dergleichen an der Wade festlegbar ist, wobei sich die Beinschale mit einem Vorfuß- Schalenabschnitt bis unter den Fußsohlenbereich erstreckt. Eine derartige Orthese ist zB aus DE-A-2 259 945 bekannt.

Zur Behandlung von frischen Sprunggelenkaußenbandläsionen wurde im Rahmen einer konservativen Versorgung bis noch vor wenigen Jahren eine anfängliche Gipsschienenruhigstellung mit Einstellung des Fußes in Pronation und Dorsalextension für ca. zwei Wochen bis zum weitgehenden Rückgang der Schwellung und eine anschließende etwa vierwöchige Unterschenkelgehgipsversorgung durchgeführt. Eine derartige Gipsbehandlung wird heute aus verschiedenen Gründen im allgemeinen als obsolet angesehen. Die konservative Versorgung ist daher heute auf eine funktionelle Behandlung ausgerichtet.

Die bisher verfügbaren Sprunggelenkorthesen umfassen nahezu ausschließlich den Rückfuß, ohne weiter zur Fußsohle zu reichen. Über dem durch Bluterguß und Gewebswasser geschwollenen, lädierten Sprunggelenksaußenbereich haben feste Schienenbestandteile oder Gurtungen ihre Anlage, wodurch diese empfindliche Region irritiert werden kann. Das Hauptproblem liegt jedoch darin, daß die Plantarflexion in der Regel nicht eingeschränkt ist. Bei Schuhversorgungen mit Sprunggelenksversteifungen bringt der Betroffene in der Regel schon beim Einschlupf in den Schuh den Fuß in Plantarflexion und schädigt damit den in Heilung begriffenen Bandapparat, insbesondere das Lig. talo-figulare anterius.

Aus funktionellen Gesichtspunkten hat sich der Tapeverband bewährt. Hiermit wird zwar ein guter Halt erreicht; nachteilig ist hingegen der Zeit- und Materialaufwand zum Anlegen des Verbandes. Weitere Nachteile ergeben sich aus der wiederholt notwendigen Erneuerung des Tapeverbandes in hygienischer Hinsicht sowie aus der Notwendigkeit, daß die Anlage des Tapeverbandes nicht nur viel Zeit, sondern vor allem auch ausreichende Erfahrung beansprucht.

Der Erfindung liegt die Aufgabe zugrunde, die eingangs beschriebene Sprunggelenkorthese hinsichtlich ihrer Funktion sowie ihres Aufbaus zu verbessern.

Diese Aufgabe wird erfindungsgemäß dadurch gelöst, daß die Beinschale im Sprunggelenkaußenbandbereich durch eine diesen Bereich freilassende Ausnehmung dorsal stark verjüngt und nach innen geführt ist, und daß ein gummielastischer Zuggurt einerseits unten außen am Vorfuß-Schalenabschnitt und andererseits oben innen im Wadenbereich an der Beinschale mit dynamischer Zugwirkung angreift, wobei die Beinschale in Dorsalextension und Pronation biegbar ist, während durch den Zuggurt Supination und Plantarflexion eingeschränkt sind.

Diese erfindungsgemäße Sprunggelenkorthese wird den Erfordernissen der funktionellen konservativen Behandlung gerecht, denn es wird eine freie Bewegung des Fußes in Dorsalextension und Pronation ermöglicht, während Plantarflexion und Supination mit dynamischer Zugwirkung durch den Zuggurt bei größer werdendem Bewegungsausmaß zunehmend eingeschränkt werden. Der Sprunggelenksaußenbandbereich ist gänzlich ausgespart.

Die Anlegung der Orthese ist besonders einfach. Dabei kann der Zuggurt zugleich auch eine um den Fuß schlingbare Vorfußfixierung bilden. Zusätzlich sind zur Festlegung nur noch eine Fußspannfixierung sowie ein Wadenband erforderlich, die bei Verwendung von Klettverschlüssen leicht anpaßbar und festlegbar sind.

Um eine Abrollung über die Metatarsaleköpfchen zu ermöglichen, ist es vorteilhaft, wenn der Vorfuß-Schalenabschnitt auch bei weiter Umfassung der Fußsohle bis unter den Vorfuß das erste Metatarsaleköpfchen freiläßt.

Während bei vorbekannten Ausführungsformen der Fuß in einer bestimmten Stellung fest fixiert wird, beispielsweise durch eine bis in den Fußsohlenbereich die Wade und den Fuß beidseitig halbschalenförmig umgreifende Beinschale, beinhaltet die erfindungsgemäße Lösung eine dynamisch wirkende Konstruktion, die bei entspannter Fußhaltung eine permanente Zugwirkung in Richtung der Therapiestellung hervorruft. Dadurch wird z. B. im "unbewußten" Zustand eines Sportlers für seinen Fuß die Idealposition gesichert, während bei Ausübung sportlicher Betätigung größtmögliche Freiheit gewährleistet ist.

Weitere Merkmale der Erfindung sind Gegenstand der Unteransprüche und werden in Verbindung mit weiteren Vorteilen der Erfindung anhand eines Ausführungsbeispieles näher erläutert.

In der Zeichnung ist eine als Beispiel dienende Ausführungsform der Erfindung dargestellt. Es zeigen:
- **Figur 1 -**: die Innenansicht einer an der Wade bzw. am Fuß festgelegten Sprunggelenkorthese und
- **Figur 2 -**: die Außenansicht der Darstellung gemäß **Figur 1**.

Die dargestellte Sprunggelenkorthese weist eine dorsale, sich über den Rückfuß erstreckende Beinschale 1 auf, die sich mit einem Vorfuß-Schalenabschnitt 2 bis unter den Fußsohlenbereich erstreckt und im Sprunggelenkaußenbandbereich 3 durch eine diesen Bereich freilassende Ausnehmung 4 dorsal stark verjüngt und nach innen geführt ist. Die erfindungsgemäße Sprunggelenkorthese umfaßt ferner einen gummielastischen Zuggurt 5, der einerseits unten außen am Vorfuß-Schalenabschnitt 2 und andererseits oben innen im Wadenbereich an der Beinschale 1 mit dynamischer Zugwirkung angreift. Der Zuggurt 5 verläuft also ventral diagonal. Die Beinschale 1 ist aufgrund ihrer Gestaltung in Dorsalextension und Pronation biegbar, während durch den Zuggurt 5 Supination und Plantarflexion eingeschänkt sind.

Der Zuggurt 5 bildet bei der dargestellten Ausführungsform zugleich auch eine um den Fuß 6 schlingbare Vorfußfixierung 7. Zur weiteren Befestigung der Sprunggelenkorthese dienen noch eine Fußspannfixierung 8 sowie ein Wadenband 9, die jeweils durch Klettverschlüsse oder dergleichen festlegbar sind.

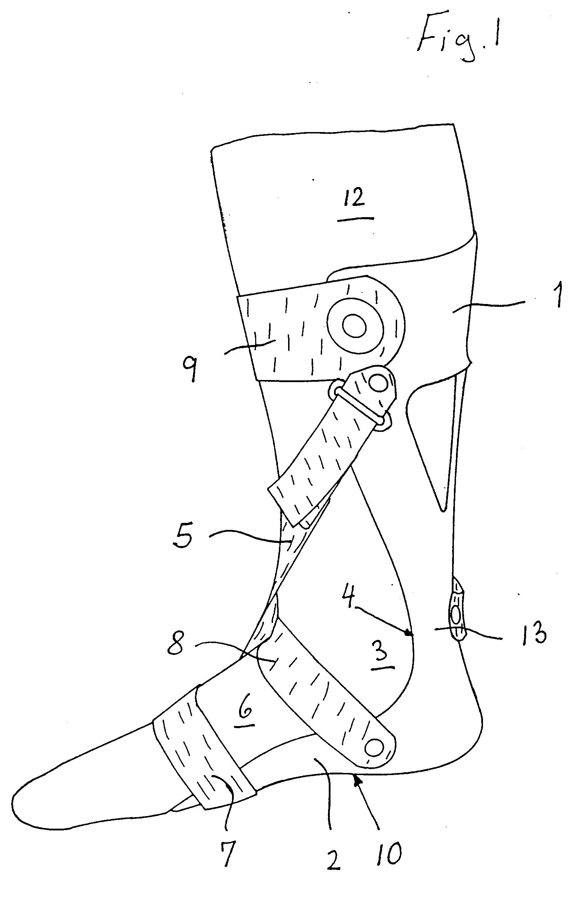

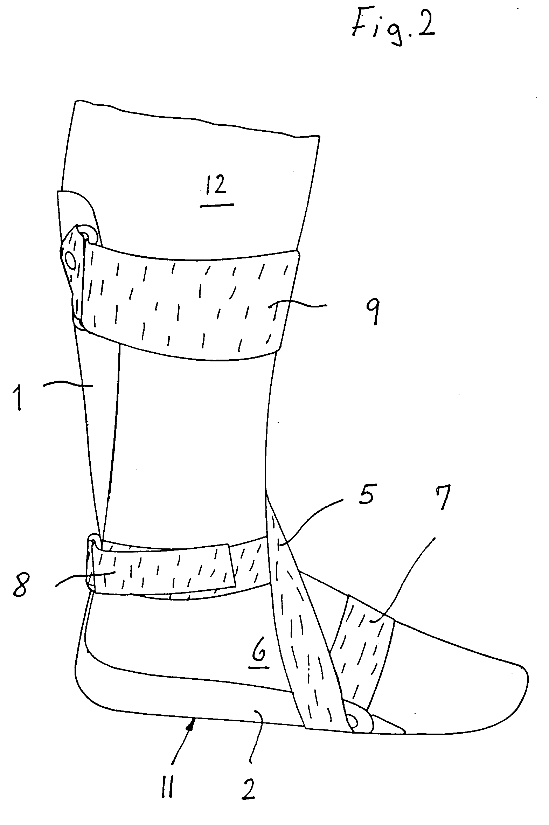

In Figur 1 kennzeichnet der Pfeil 10 den Fußinnenrand, während in Figur 2 der Pfeil 11 den Fußaußenrand kennzeichnet. Dabei macht ein Vergleich der Figuren 1 und 2 miteinander deutlich, daß die Beinschale 1 die Wade 12 nur dorsal und innenseitig umgreift, die Außenseite der Wade 12 jedoch weitgehend freiläßt. Aufgrund der großen, im Sprunggelenkaußenbandbereich 3 vorgesehenen Ausnehmung 4 ergibt sich zum Vorfuß-Schalenabschnitt 2 ein verhältnismäßig schmal ausgebildeter, weitgehend auf der Fußinnenseite liegender Schalenverbindungsbereich 13, der biegeelastisch ausgebildet ist und der Beinschale 1 eine Eigenspannung verleiht.

Die Beinschale 1 besteht vorzugsweise aus einem thermoplastischen Kunststoff und ist daher nachformbar und somit individuell anpaßbar. Sie kann in einem Schuh getragen werden.

## Patentansprüche

1. Sprunggelenkorthese mit einer dorsalen, sich über den Rückfuß erstreckenden Beinschale (1), die über Gurtungen oder dergleichen an der Wade (12) festlegbar ist, wobei sich die Beinschale mit einem Vorfuß-Schalenabschnitt (2) bis unter den Fußsohlenbereich erstreckt, **dadurch gekennzeichnet**, daß die Beinschale (1) im Sprunggelenkaußenbandbereich (3) durch eine diesen Bereich freilassende Ausnehmung (4) dorsal stark verjüngt und nach innen geführt ist, und daß ein gummielastischer Zuggurt (5) einerseits unten außen am Vorfuß-Schalenabschnitt (2) und andererseits oben innen im Wadenbereich an der Beinschale (1) mit dynamischer Zugwirkung angreift, wobei die Beinschale (1) in Dorsalextension und Pronation biegbar ist, während durch den Zuggurt (5) Supination und Plantarflexion eingeschränkt sind.

2. Sprunggelenkorthese nach Anspruch 1, **dadurch gekennzeichnet**, daß der Zuggurt (5) zugleich auch eine um den Fuß (6) schlingbare Vorfußfixierung (7) bildet.

3. Sprunggelenkorthese nach Anspruch 2, **dadurch gekennzeichnet**, daß die genannten Gurtungen oder dergleichen neben der Vorfußfixierung (7) nur noch eine Fußspannfixierung (8) sowie ein Wadenband (9) umfassen.

4. Sprunggelenkorthese nach Anspruch 1, 2 oder 3, **dadurch gekennzeichnet**, daß der Vorfuß-Schalenabschnitt (2) auch bei weiter Umfassung der Fußsohle bis unter den Vorfuß das erste Metatarsaleköpfchen freiläßt.

## Claims

1. Ankle joint orthosis, having a dorsal leg shell (1) which extends over the rear of the foot and which can be fixed to the calf (12) by way of belts or the like, the leg shell extending by means of a shell section (2) for the front of the foot as far as under the sole region of the foot, characterized in that the leg shell (1) is dorsally sharply tapered and is guided inward in the ankle joint outer ligament region (3) by means of a cutout (4) leaving this region free, and in that a rubber-elastic tensioning belt (5) acts on the one hand at the bottom on the outside on the shell section (2) for the front of the foot and acts on the other hand at the top on the inside in the calf region on the leg shell (1) with dynamic tensioning action, the leg shell (1) being bendable in dorsal extension and pronation, while supination and plantar flexion are restricted by the tensioning belt (5).

2. Ankle joint orthosis according to Claim 1, characterized in that the tensioning belt (5) at the same time also forms a means (7) for fixing the front of the foot which can be wrapped around the foot (6).

3. Ankle joint orthosis according to Claim 2, characterized in that the said belts or the like, in addition to the means (7) for fixing the front of the foot, also comprise a means (8) for fixing the instep as well as a calf strap (9).

4. Ankle joint orthosis according to Claim 1, 2 or 3, characterized in that, even when it widely embraces the sole of the foot as far as under the front of the foot, the shell section (2) for the front of the foot leaves the first metatarsal head free.

## Revendications

1. Orthèse de l'articulation de la cheville, avec une coquille dorsale de jambe (1) s'étendant au-dessus de l'arrière du pied, qui peut être fixée sur le mollet (12) par l'intermédiaire de sangles ou similaires, la coquille de jambe s'étendant par un tronçon de coquille d'avant-pied (2) jusqu'en dessous de la zone de la plante du pied, caractérisée en ce que la coquille de jambe (1) se rétrécit fortement en direction dorsale dans la zone des ligaments externes de l'articulation de la cheville (3), par une découpe (4) laissant libre cette zone, et est guidée vers l'intérieur, et en ce qu'une sangle (5) élastique en caoutchouc s'accroche avec un effet de traction dynamique, d'une part, en dessous et à l'extérieur au tronçon de coquille d'avant-pied (2) et, d'autre part, vers le haut et l'intérieur à la coquille de jambe (1) dans la zone du mollet, la coquille de jambe (1) étant flexible en extension dorsale et en pronation, tandis que, grâce à la sangle de traction (5), la supination et la flexion plantaire sont restreintes.

2. Orthèse de l'articulation de la cheville selon la revendication 1, caractérisée en ce que la sangle de traction (5) forme en même temps une fixation (7) de l'avant-pied pouvant être enroulée autour du pied (6).

3. Orthèse de l'articulation de la cheville selon la revendication 2, caractérisée en ce qu'en plus de la fixation de l'avant-pied (7), lesdites sangles ou similaires comprennent uniquement une fixation tendue du pied (8) ainsi qu'une sangle de mollet (9).

4. Orthèse de l'articulation de la cheville selon la revendication 1, 2 ou 3, caractérisée en ce que même lorsqu'il entoure la plante du pied plus loin jusqu'en dessous de l'avant-pied, le tronçon de coquille d'avant-pied (2) laisse libre la première tête métatarsienne.
